# EUROPEAN PATENT SPECIFICATION

(11) **EP 4 631 415 B1**
(45) Date of publication and mention of the grant of the patent: **21.10.2026**
(21) Application number: 24777788.1
(22) Date of filing: 19.03.2024
(51) Int. Cl.: A61B 1/00, A61B 10/00, A61M 39/22, A61B 1/015, A61B 10/04, B01L 3/00

(54) **CONTROL VALVE FOR SAMPLE COLLECTION CONTAINER, AND SAMPLE COLLECTION CONTAINER AND ENDOSCOPE**
STEUERVENTIL FÜR PROBENSAMMELBEHÄLTER SOWIE PROBENSAMMELBEHÄLTER UND ENDOSKOP
VANNE DE COMMANDE POUR RÉCIPIENT DE COLLECTE D'ÉCHANTILLON, ET RÉCIPIENT DE COLLECTE D'ÉCHANTILLON ET ENDOSCOPE

(30) Priority: 31.03.2023 CN 202310335658
(43) Date of publication of application: 15.10.2025
(73) Proprietor: Hunan Vathin Medical Instrument Co., Ltd., Xiangtan, Hunan 411100 (CN)
(72) Inventor: ZHOU, Zhenhua, Xiangtan, Hunan 411100 (CN)
(74) Representative: Bayramoglu et al.
(86) International application number: PCT/CN2024/082368
(87) International publication number: WO 2024/198999

(56) References cited:
- CN-A- 105 179 742
- CN-A- 110 047 665
- CN-A- 110 251 050
- CN-A- 116 269 158
- CN-U- 204 784 849
- CN-U- 218 165 295
- CN-U- 218 582 336
- CN-Y- 201 116 610
- JP-A- 2002 228 025
- JP-A- 2019 148 317
- US-A1- 2019 254 505

## Description

### TECHNICAL FIELD

The present disclosure relates to the technical field of endoscopy, and in particular to a control valve for a specimen collection container, a specimen collection container, and an endoscope.

### BACKGROUND TECHNOLOGY

The endoscope is a critical medical device in modern medical technology. It can be used in conjunction with a specimen bottle by docking the specimen bottle to the endoscope handle. Thus, the inserting unit of the endoscope advances into the body cavity for sampling, and the specimen is channeled through the endoscope into the specimen bottle for collection.

During the implementation of the present disclosure, it has been determined that incorporating a control valve into the docking structure between the specimen bottle and the endoscope handle is essential to ensure effective collection of a target specimen. The control valve can selectively direct the target specimen into the specimen bottle at an appropriate sampling point and direct the specimen into a waste bottle at a non-sampling point. That is, the control valve is required to switch specimen flow paths between the specimen bottle and the waste bottle. However, leakage may occur during the flow path switching operation of the control valve, thereby compromising use convenience. CN110251050A discloses an attraction seat which comprises a valve body, a valve element, a first opening, a second opening and a third opening. The first opening and the second opening are used for liquid incoming and outgoing, an inside runner of the valve body is larger than the middle of the valve element, a groove along the axial direction of the valve element is formed in the peripheral wall of the middle of the valve element, the lower end of the groove is close to a sealing flange, the third opening is used for pressing the upper end of the valve element and enabling the lower end of the groove to extend out of the first opening, a first magnet is downwards abutted against the valve body, and the second magnet is upwards abutted against the valve element to enable the same to upwards reset and the sealing flange to seal the first opening. Two magnetic rings are adopted to replace a flexible springback material.

### CONTENT OF THE INVENTION

An objective of the present disclosure is to provide a control valve for a specimen collection container, a specimen collection container, and an endoscope. The present disclosure can solve the above technical problems in the prior art, and mainly includes the following three aspects.

A first aspect of the present disclosure provides a control valve for a specimen collection container, including:
a valve body, where the valve body is provided with an input passage and an output passage; the valve body is internally provided with a mounting cavity; and the input passage and the output passage communicate with the mounting cavity separately;
a main seal, where the main seal is an annular structure; the main seal is fixedly connected to an inner wall of the mounting cavity; and the main seal is provided with a through hole communicating with the input passage correspondingly; and
a valve spool, where the valve spool is at least partially provided inside the main seal; a rotational stroke exists between the valve spool and the main seal; the valve spool is provided with a communication passage; the communication passage is configured to enable communication between the through hole and the output passage; a side wall of the main seal adjacent to the valve spool is provided with an annular sub-seal; and the sub-seal is configured to enable sealed communication between the communication passage and the through hole;
and a balance member, where the balance member is provided on the valve body; and the balance member and the sub-seal abut against two sides of the valve spool respectively to maintain the valve spool coaxial with the main seal during the rotational stroke.

Furthermore, preferably, the valve body is provided with a rotary control cavity communicating with the mounting cavity; a rotary control member is provided in the rotary control cavity; and the rotary control member is connected to the valve spool.

Furthermore, preferably, a sliding groove is provided on a peripheral wall of the rotary control member; the balance member is provided on an inner wall of the rotary control cavity; the balance member abuts against the valve spool via the rotary control member; and the balance member is in a sliding fit with the sliding groove.

Furthermore, preferably, a mounting groove matched with the balance member is provided on the peripheral wall of the rotary control member; the mounting groove is provided along an axial direction of the rotary control cavity; and the mounting groove communicates with the sliding groove.

Furthermore, preferably, a stop member is provided in the sliding groove; and the stop member is configured to limit a sliding stroke of the balance member in the sliding groove to control the rotational stroke of the valve spool.

Furthermore, preferably, the output passage includes a first output sub-passage and a second output sub-passage; the first output sub-passage is configured to be connected to a bottle body; and the second output sub-passage is configured to be connected to a negative pressure source;
the communication passage includes a first communication sub-passage, a second communication sub-passage, and a third communication sub-passage;
the through hole includes a first sub-hole and a second sub-hole; and
the rotational stroke of the valve spool includes a first position and a second position; in the first position, the input passage, the first sub-hole, the first communication sub-passage, and the first output sub-passage communicate sequentially, and the first output sub-passage, the second communication sub-passage, the second sub-hole, and the second output sub-passage communicate sequentially; and in the second position, the input passage, the first sub-hole, the third communication sub-passage, the second sub-hole, and the second output sub-passage communicate sequentially.

Furthermore, preferably, the communication passage is located above the first output sub-passage.

Furthermore, preferably, the valve spool is provided with a sealing surface; the sealing surface is configured to isolate the first output sub-passage from the mounting cavity; and an output port of the first communication sub-passage and an input port of the second communication sub-passage are located on the sealing surface.

A second aspect of the present disclosure provides a specimen collection container, including a bottle body and the above-mentioned control valve, where the control valve is detachably connected to the bottle body; and the output passage communicates with an interior of the bottle body.

A third aspect of the present disclosure provides an endoscope, including a handle, where the handle is provided with a specimen extraction port; the endoscope further includes the above-mentioned control valve or the above-mentioned specimen collection container; and the specimen extraction port communicates with the input passage.

Compared with the prior art, the present disclosure has at least the following technical effects.

**In** the present disclosure, the sub-seal improves the sealing performance. Meanwhile, the balance member provided on the valve body cooperates with the sub-seal to maintain the valve spool coaxial with the seal during the rotational stroke. At this point, the contact between the valve spool and the main seal is concentrated at the sub-seal. Due to the small contact area corresponding to the sub-seal, the frictional resistance exerted by the main seal on the valve spool during rotation is significantly reduced. Thus, the present disclosure reduces the driving force required for the control valve during flow path switching control while improving its sealing performance, thereby enhancing operational convenience and safety of the device.

### DESCRIPTION OF THE DRAWINGS

To describe the technical solutions in the embodiments of the present disclosure more clearly, the following briefly introduces the drawings required for describing the embodiments or the prior art. Apparently, the drawings in the following description show merely some embodiments of the present disclosure, and persons of ordinary skill in the art may still derive other drawings from these drawings without creative efforts.
FIG. 1 is a structural schematic diagram of a control valve connected to a bottle body according to the present disclosure;
FIG. 2 is a structural schematic diagram of FIG. 1 from another perspective according to the present disclosure;
FIG. 3 is an internal structural schematic diagram of the control valve according to the present disclosure;
FIG. 4 is an exploded view of a specimen collection container according to the present disclosure;
FIG. 5 is a top view of the specimen collection container according to the present disclosure; and
FIG. 6 is a sectional view taken along A-A shown in FIG. 5;
FIG. 7 is a structural schematic diagram of a valve spool according to the present disclosure;
FIG. 8 is a structural schematic diagram of a main seal according to the present disclosure;
FIG. 9 is a front view of the main seal according to the present disclosure;
FIG. 10 is a schematic diagram of the valve spool connected to the main seal (in a first position) according to the present disclosure;
FIG. 11 is a sectional view taken along B-B shown in FIG. 10;
FIG. 12 is a schematic diagram of the valve spool connected to the main seal (in a second position) according to the present disclosure;
FIG. 13 is a structural schematic diagram of a rotary control member according to the present disclosure;
FIG. 14 is a schematic diagram of specimen flow in the specimen collection container at the first position according to the present disclosure; and
FIG. 15 is a structural schematic diagram of an endoscope according to the present disclosure.

### Reference Numerals:

100. valve body; 110. input passage; 120. output passage; 121. first output sub-passage; 122. second output sub-passage; 130. mounting cavity; 140. balance member; 200. valve spool; 210. communication passage; 211. first communication sub-passage; 212. second communication sub-passage; 213. third communication sub-passage; 220. sealing surface; 300. main seal; 310. through hole; 311. first sub-hole; 312. second sub-hole; 320. sub-seal; 400. rotary control member; 410. sliding groove; 420. stop member; 430. mounting groove; 500. bottle body; 600. handle; and 610. specimen extraction port.

### SPECIFIC IMPLEMENTATIONS

The following description provides many different embodiments or examples for implementing different features of the present disclosure. The elements and arrangements described in the following specific examples are only intended to concisely express the present disclosure, and are only for illustration purposes, rather than to limit the present disclosure.

In order to make the objectives, technical solutions, and advantages of the embodiments of the present disclosure clearer, the technical solutions in the embodiments of the present disclosure will be clearly and completely described below in conjunction with the drawings in the embodiments of the present disclosure. Obviously, the described embodiments are some, rather than all of the embodiments of the present disclosure. On the basis of the embodiments of the present disclosure, all other embodiments obtained by a person of ordinary skill in the art without making creative efforts shall fall within the protection scope of the present disclosure. Therefore, the detailed description of the embodiments of the present disclosure in the drawings is not intended to limit the protection scope of the present disclosure, but merely represent the selected embodiments of the present disclosure. On the basis of the embodiments of the present disclosure, all other embodiments obtained by a person of ordinary skill in the art without making creative efforts shall fall within the protection scope of the present disclosure.

In the present disclosure, unless otherwise clearly specified, the terms "installation", "interconnection", "connection" and "fixation" etc. are intended to be understood in a broad sense. For example, the "connection" may be a fixed connection, removable connection or integral connection; may be a mechanical connection or electrical connection; may be a direct connection or indirect connection using a medium; and may be a communication or interaction between two elements. Those of ordinary skill in the art may understand specific meanings of the above terms in the present disclosure based on a specific situation. In addition, the terms such as "first", "second", and "third" are used only for the purpose of description and cannot be understood to indicate or imply relative importance.

In the present disclosure, unless otherwise expressly specified, when it is described that a first feature is "above" or "under" a second feature, it may indicate that the first feature is in direct contact with the second feature, or that the first feature and the second feature are not in direct contact with each other but are in contact via another feature between them. Moreover, "a first feature is above and on a second feature" includes "the first feature is directly above or obliquely above the second feature" or simply means that "the first feature is higher than the second feature". "A first feature is under and below a second feature" includes "the first feature is directly under or obliquely under the second feature" or simply means that "the first feature is lower than the second feature".

In addition, in the present disclosure, for the convenience of describing and understanding the positional relationship between components, "proximal end" and "distal end" refer to proximal and distal positions of a structure for in-vivo operation in an operating environment. For the same component, "proximal end" and "distal end" refer to the relative rather than absolute positional relationship of the component. Therefore, the understanding of "proximal end" and "distal end" should be based on the principles of the present disclosure, without deviating from the essence of the present disclosure.

### Embodiment 1

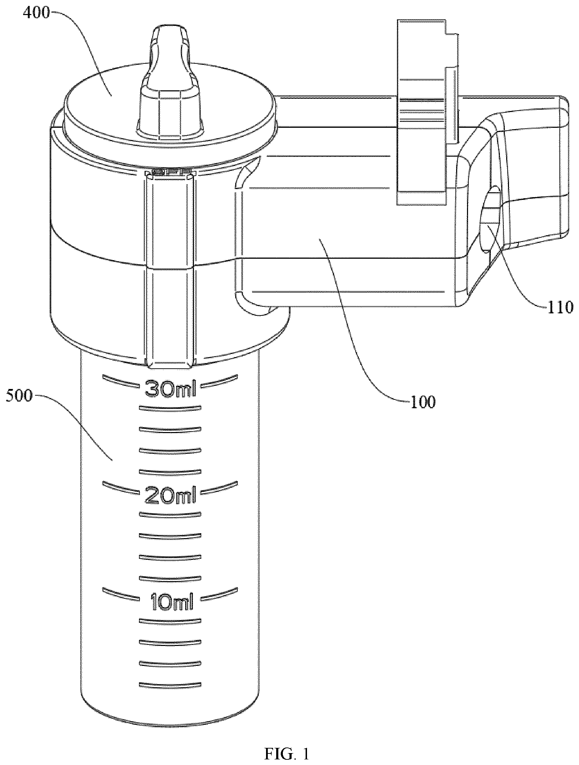

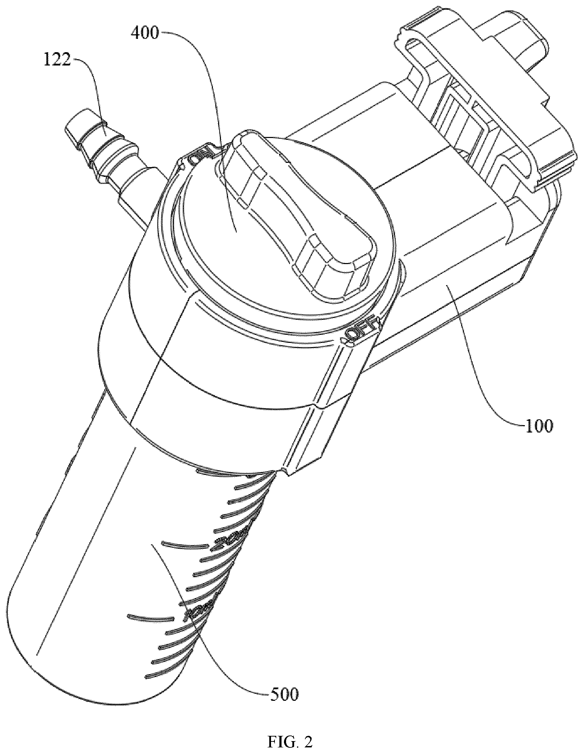

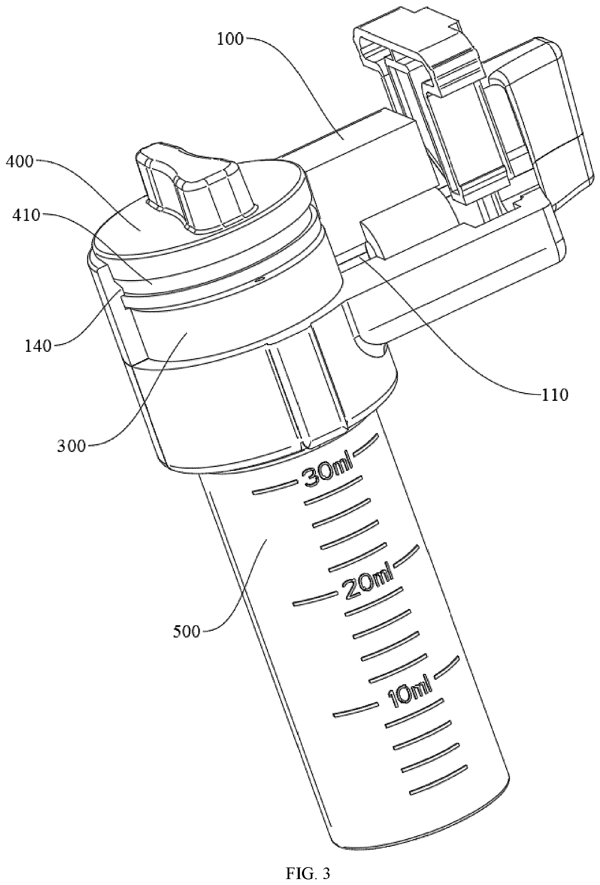

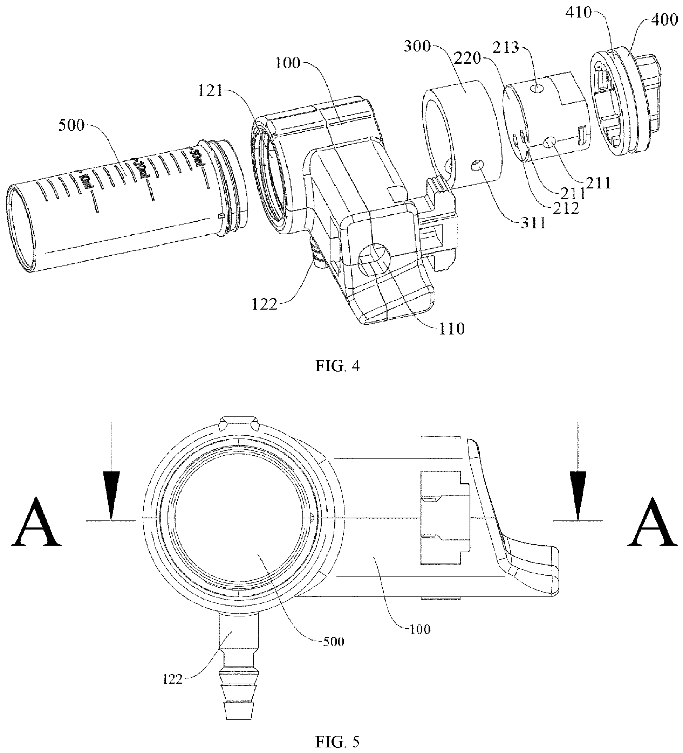

The embodiment of the present disclosure provides a control valve for a specimen collection container. As shown in FIG. 1 to FIG. 6, the control valve includes: valve body 100, main seal 300, valve spool 200, and balance member 140.

The valve body 100 is provided with input passage 110 and output passage 120. The valve body 100 is internally provided with mounting cavity 130. The input passage 110 and the output passage 120 communicate with the mounting cavity 130 separately.

The main seal 300 is an annular structure. The main seal 300 is fixedly connected to an inner wall of the mounting cavity 130. The main seal 300 is provided with through hole 310 communicating with the input passage 110 correspondingly.

The valve spool 200 is at least partially provided inside the main seal 300. A rotational stroke exists between the valve spool 200 and the main seal 300. The valve spool 200 is provided with communication passage 210. The communication passage 210 is configured to enable communication between the through hole 310 and the output passage 120. A side wall of the main seal 300 adjacent to the valve spool 200 is provided with annular sub-seal 320. The sub-seal 320 is configured to enable sealed communication between the communication passage 210 and the through hole 310.

The balance member 140 is provided on the valve body 100. The balance member 140 and the sub-seal 320 abut against two sides of the valve spool 200 respectively to maintain the valve spool 200 coaxial with the main seal 300 during the rotational stroke.

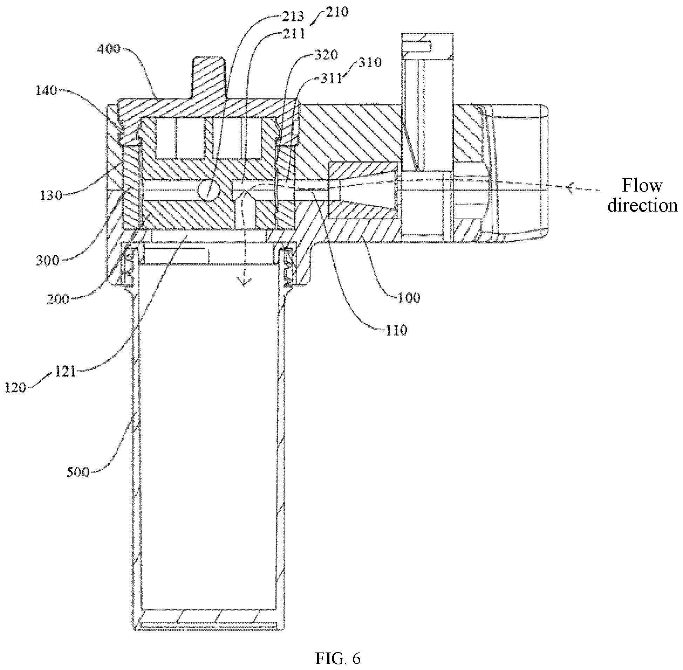

To achieve effective collection of a target specimen, the control valve is provided on a docking structure between a specimen bottle and an endoscope handle. Under a differential pressure, the control valve selectively directs the target specimen into the specimen bottle at an appropriate sampling point and directs the specimen to a waste bottle at a non-sampling point. That is, the control valve is design to switch specimen flow paths between the specimen bottle and the waste bottle. Therefore, the control valve is generally provided with two directing passages, one of which communicates with the specimen bottle and the other communicates with the waste bottle. Meanwhile, to improve the sealing performance of the control valve, the seal is generally provided between the valve body 100 and the valve spool 200 of the control valve. To facilitate flow path switching operation, the driving force requirement for performing flow path switching needs to be reduced. In prior art, the rotational frictional resistance of the valve spool 200 is typically reduced by adjusting the fitting tightness between the valve spool 200 and the seal and decreasing the contact pressure between the valve spool 200 and the seal. When the contact pressure between the valve spool 200 and the seal is small, under a differential pressure condition, the communication point between the valve spool 200 and the seal is highly prone to leakage due to the differential pressure. Consequently, in the prior art, the control valve is prone to leakage under a low driving force condition. In the embodiment, as shown in FIG. 6, the main seal 300 provides primary sealing between the valve spool 200 and the valve body 100. To further ensure the communication sealing between the valve spool 200 and the valve body 100, the sub-seal 320 is provided on the main seal 300 to perform targeted sealing on the communication port between the communication passage 210 and the through hole 310. This further enhances the sealing performance of the control valve. Meanwhile, the balance member 140 is provided on the valve body 100 to cooperate with the sub-seal 320, maintaining the valve spool 200 coaxial with the main seal 300 during the rotational stroke. At this point, the contact between the valve spool 200 and the main seal 300 is concentrated at the sub-seal 320. Due to the small contact area corresponding to the sub-seal 320, the frictional resistance exerted on the valve spool 200 by the main seal 300 during rotation is significantly reduced. Thus, the present disclosure reduces the driving force required for the control valve during flow path switching control while improving its sealing performance, thereby enhancing operational convenience and safety of the device. Additionally, the balance member 140 is added to the valve body 100. The balance member 140 cooperates with the sub-seal 320 to effectively suppress spatial occupancy impacts caused by the sub-seal 320 and prevent deviation of the valve spool 200 from the central axis of the valve body 100. It should be noted that if the valve spool 200 deviates from the central axis of the valve body 100, the contact pressures between the valve spool 200 (the two sides of the valve spool 200 corresponding to the sub-seal 320) and the main seal 300 will differ. Specifically, the sealing contact pressure between the side of the main seal 300 without the sub-seal 320 and the valve spool 200 is small. Under differential pressure conditions, this side is highly susceptible to pressure breakthrough during flow path switching, causing specimen leakage. The cooperation between the balance member 140 and the sub-seal 320 ensures that the control valve meets the low driving force requirement while further improving its sealing performance.

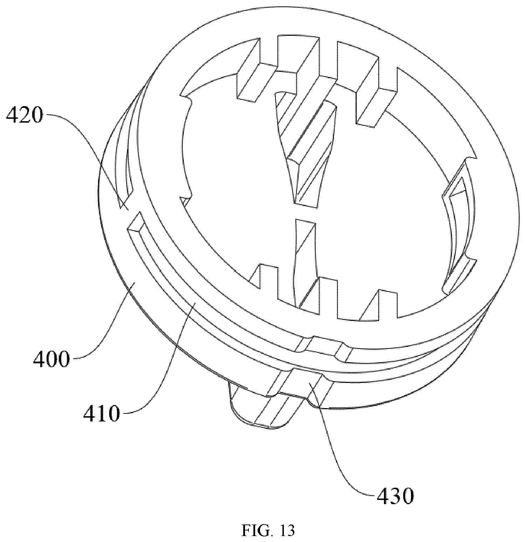

Specifically, to facilitate the control of an operator over the rotation of the valve spool 200 relative to the main seal 300, a rotary control cavity communicating with the mounting cavity 130 is provided on the valve body 100, and rotary control member 400 is provided in the rotary control cavity. As shown in FIG. 13, the rotary control member 400 is connected to the valve spool 200. The connection between the rotary control member 400 and the valve spool 200 may specifically be a snap connection, a mortise-and-tenon connection, a threaded connection, a bolted connection, or an interference fit, which is not specifically limited herein. Furthermore, to facilitate the operator to exert force on the rotary control member 400, a protruding portion or an additional friction layer is provided on the rotary control member 400 to transmit power to the rotary control member 400 more efficiently.

Specifically, to enable relatively stable rotation of the valve spool 200 within the main seal 300, sliding groove 410 is provided on a peripheral wall of the rotary control member 400. The balance member 140 is provided on an inner wall of the rotary control cavity, enabling a sliding fit between the balance member 140 and the sliding groove 410. The design can constrain the positions of the valve spool 200 and the rotary control member 400 relative to the valve body 100 during the rotational stroke, maintaining structural stability and balance of the control valve during rotary control. Meanwhile, the balance member 140 abuts against the rotary control member 400, and the abutting force is transmitted to the valve spool 200 through the rotary control member 400 to cooperate with the sub-seal 320. Thus, the valve spool 200 always remains coaxial within the main seal 300, and a small contact area is maintained between the valve spool 200 and the main seal 300, reducing the frictional resistance exerted on the valve spool 200 by the main seal 300 during rotation.

In some embodiments, to reduce frictional resistance between the valve spool 200 and the balance member 140, a sliding surface is provided on the balance member 140 and/or the sliding groove 410. The sliding surface can reduce the contact area between the balance member 140 and the sliding groove 410 or lower the friction coefficient between the balance member 140 and the sliding groove 410.

Specifically, to facilitate mounting of the rotary control member 400 into the rotary control cavity, mounting groove 430 matched with the balance member 140 is provided on the peripheral wall of the rotary control member 400. The mounting groove 430 is provided along an axial direction of the rotary control cavity. When the rotary control member 400 is pushed into the rotary control cavity along the axial direction, the balance member 140 corresponding to the mounting groove 430 can freely pass through the mounting groove 430. The mounting groove 430 provides clearance for the balance member 140, ensuring no interference between the balance member 140 and the rotary control member 400 during mounting. The mounting groove 430 communicates with the sliding groove 410. After the rotary control member 400 is mounted in place, the balance member 140 is precisely located at a communication point in a junction zone between the mounting groove 430 and the sliding groove 410. The rotary control member 400 is rotated circumferentially along the rotary control cavity, causing the balance member 140 to enter the sliding groove 410. The design achieves the sliding fit between the balance member 140 and the rotary control member 400, and allows the balance member 140 to constrain the rotary control member 400 within the rotary control cavity. The design simplifies the structure of the control valve, reduces manufacturing costs and assembly difficulty of the control valve, and improves spatial utilization of the control valve.

Specifically, to facilitate effective control of the operator over the rotational stroke of the valve spool 200, stop member 420 is provided in the sliding groove 410. The stop member 420 limits a sliding stroke of the balance member 140 in the sliding groove 410 to achieve control over the rotational stroke of the valve spool 200. Thus, the operator can perceive the upper and lower limits of the rotational adjustment stroke of the valve spool 200 through resistance feedback from the stop member 420 against the balance member 140, thereby improving accuracy of flow path switching control performed by the operator.

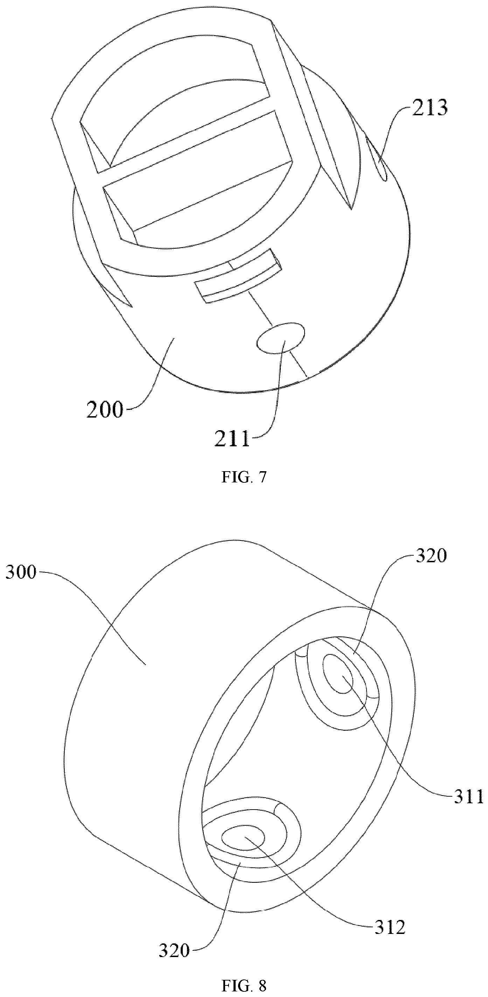

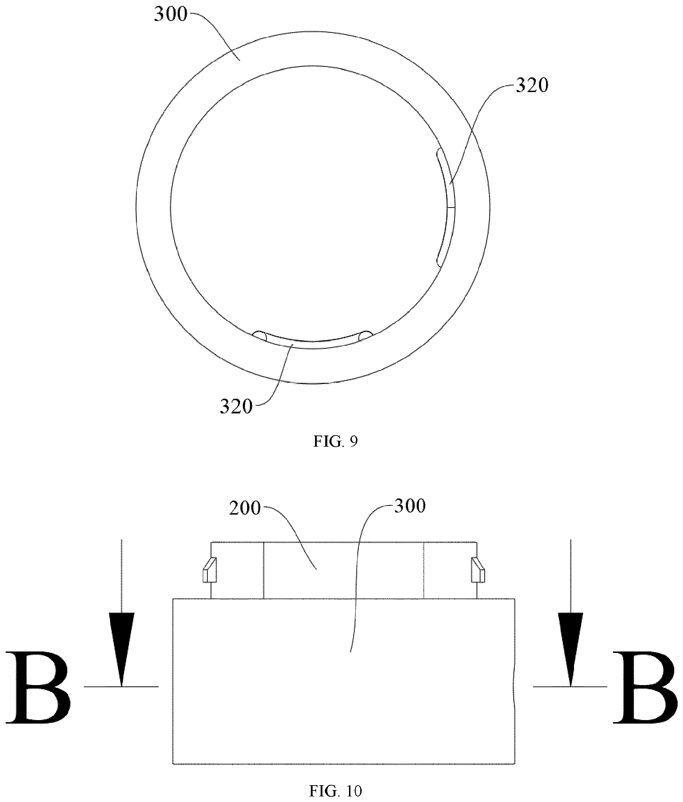

Specifically, as shown in FIG. 6 to FIG. 12, to achieve flow path switching between the specimen bottle and the waste bottle by the control valve, the output passage 120 includes first output sub-passage 121 and second output sub-passage 122. The first output sub-passage 121 is configured to be connected to bottle body 500 (specifically, the specimen bottle). The second output sub-passage 122 is configured to be connected to a negative pressure source (specifically, the second output sub-passage 122 communicates with the negative pressure source via the waste bottle).

The communication passage 210 includes first communication sub-passage 211, second communication sub-passage 212, and third communication sub-passage 213.

The through hole 310 includes first sub-hole 311 and second sub-hole 312.

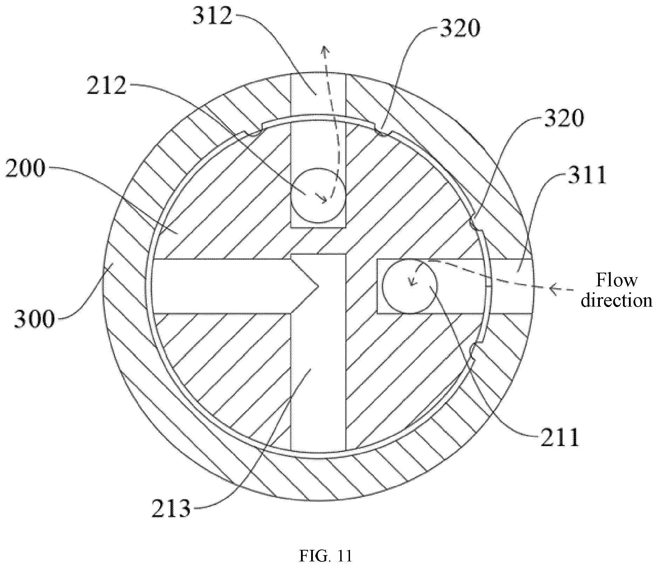

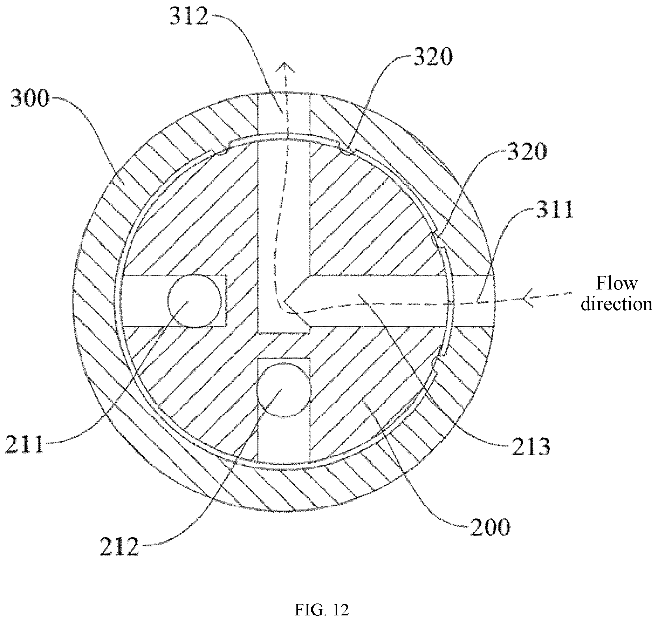

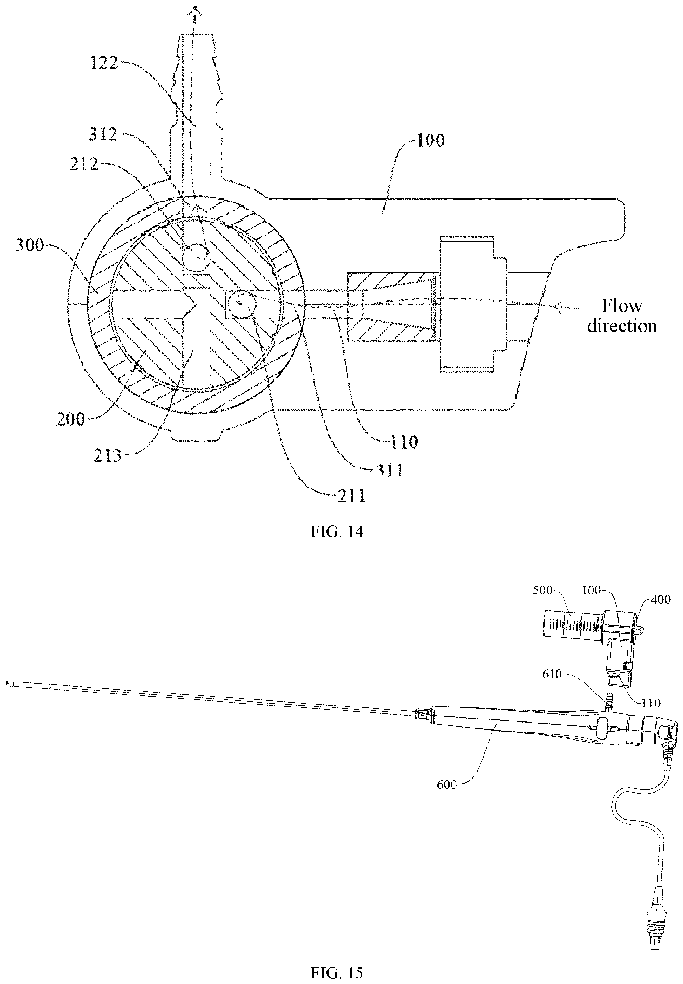

The rotational stroke of the valve spool 200 includes a first position and a second position. In the first position, as shown in FIG. 11 and FIG. 14, the input passage 110, the first sub-hole 311, the first communication sub-passage 211, and the first output sub-passage 121 communicate sequentially. The first output sub-passage 121, the second communication sub-passage 212, the second sub-hole 312, and the second output sub-passage 122 communicate sequentially. In the second position, as shown in FIG. 12, the input passage 110, the first sub-hole 311, the third communication sub-passage 213, the second sub-hole 312, and the second output sub-passage 122 communicate sequentially.

Based on this, when it is required to direct the specimen into the bottle body 500 for collection, the valve spool 200 is rotated to the first position. At this point, the differential pressure between the negative pressure source and an inlet end of the input passage 110 serves as the driving energy for specimen flow. The specimen can pass sequentially through the input passage 110, the first sub-hole 311, the first communication sub-passage 211, and the first output sub-passage 121. After entering the first output sub-passage 121, the specimen falls into the bottle body 500 under gravity. Since the bottle body 500, the first output sub-passage 121, the second communication sub-passage 212, the second sub-hole 312, and the second output sub-passage 122 communicate sequentially, the bottle body 500 maintains a lower pressure relative to the inlet end of the input passage 110. Thus, the specimen continuously flows from the input passage 110 into the bottle body 500 to achieve specimen collection. When it is unnecessary to direct the specimen into the bottle body 500, the valve spool 200 is rotated to the second position. At this point, driven by the differential pressure, the specimen passes sequentially through the input passage 110, the first sub-hole 311, the third communication sub-passage 213, the second sub-hole 312, and the second output sub-passage 122, flowing toward the negative pressure source. During the flow of the specimen toward the negative pressure source, the specimen is directed into the waste bottle for containment (this process is not the focus of the present disclosure and will not be detailed here). Thus, flow path switching between the specimen bottle and the waste bottle by the control valve is achieved. Throughout the flow path switching control process, stable high sealing is maintained among all passages of the control valve, and the frictional resistance exerted on the valve spool 200 by the main seal 300 during rotation is extremely low. Therefore, the present disclosure enables low driving force operation of the control valve, improving its operational convenience.

In some embodiments, to ensure stable rotation of the valve spool 200 to the first position and the second position by the operator, two stop members 420 can be provided in the sliding groove 410. When the balance member 140 abuts against first stop member 420, the valve spool 200 is in the first position. When the balance member 140 abuts against second stop member 420, the valve spool 200 is in the second position. Thus, the operator can promptly determine whether the valve spool 200 is rotated to the target position through resistance feedback from the rotation of the rotary control member 400, enhancing the operational convenience of the control valve.

Specifically, during specimen collection, to ensure stable entry of the specimen into the bottle body 500, the communication passage 210 is located above the first output sub-passage 121, and the bottle body 500 communicating with the first output sub-passage 121 is correspondingly located below the communication passage 210. After entering the communication passage 210 from the input passage 110, the specimen flows into the bottle body 500 under gravity to complete collection. Additionally, influenced by the negative pressure source, the bottle body 500 maintains a lower pressure relative to the inlet end of the input passage 110.

Specifically, to ensure continuous and stable inflow of the specimen into the first output sub-passage 121, sealing surface 220 is provided on the valve spool 200. The first output sub-passage 121 and the mounting cavity 130 are isolated by the sealing surface 220. Specifically, the sealing surface 220 abuts against an inlet end surface of the first output sub-passage 121 to achieve sealing isolation. An output port of the first communication sub-passage 211 and an input port of the second communication sub-passage 212 are located on the sealing surface 220. The design prevents air communication between the first output sub-passage 121 and the mounting cavity 130, ensuring the first output sub-passage 121 remains at a lower pressure relative to the inlet end of the input passage 110 during specimen collection.

### Embodiment 2

The embodiment of the present disclosure provides a specimen collection container, as shown in FIG. 1 to FIG. 14, including bottle body 500 and the control valve in Embodiment 1. The control valve is detachably connected to the bottle body 500, and the output passage 120 communicates with an interior of the bottle body 500.

During use, the bottle body 500 communicates with the output passage of the control valve. Specifically, the bottle body 500 communicates with the first output sub-passage 121. The input passage 110 of the control valve is docked with specimen extraction port 610 of endoscope handle 600. When the valve spool 200 is rotated to the first position, the specimen extraction port 610, the input passage 110, and the bottle body 500 communicate. Thus, the specimen can be directed through the specimen extraction port 610 into the bottle body 500 for collection.

### Embodiment 3

The embodiment of the present disclosure provides an endoscope, as shown in FIG. 15, including handle 600. The handle 600 is provided with specimen extraction port 610. The endoscope further includes the control valve in Embodiment 1 or the specimen collection container in Embodiment 2. The specimen extraction port 610 communicates with the input passage 110.

It should be noted that the endoscope in the embodiment of the present disclosure may be a bronchoscope, pyeloscope, esophagoscope, gastroscope, enteroscope, otoscope, nasoscope, oral endoscope, laryngoscope, colposcope, laparoscope, arthroscope, etc. The present disclosure imposes no specific limitations on the type of the endoscope.

## Claims

1. A control valve for a specimen collection container, comprising:
a valve body (100), wherein the valve body (100) is provided with an input passage (110) and an output passage (120); the valve body (100) is internally provided with a mounting cavity (130); and the input passage (110) and the output passage (120) communicate with the mounting cavity (130) separately;
a main seal (300), wherein the main seal (300) is an annular structure; the main seal (300) is fixedly connected to an inner wall of the mounting cavity (130); and
a valve spool (200), wherein the valve spool (200) is at least partially provided inside the main seal (300); and
a rotational stroke exists between the valve spool (200) and the main seal (300); and
the valve spool (200) is provided with a communication passage (210);
**characterised in that** the main seal (300) is provided with a through hole (310) communicating with the input passage (110) correspondingly; and the communication passage (210) is configured to enable communication between the through hole (310) and the output passage (120); a side wall of the main seal (300) adjacent to the valve spool (200) is provided with an annular sub-seal (320); and the sub-seal (320) is configured to enable sealed communication between the communication passage (210) and the through hole (310); and
the control valve further comprises a balance member (140), wherein the balance member (140) is provided on the valve body (100); and the balance member (140) and the sub-seal (320) abut against two sides of the valve spool (200) respectively to maintain the valve spool (200) coaxial with the main seal (300) during the rotational stroke.

2. The control valve according to claim 1, wherein the valve body (100) is provided with a rotary control cavity communicating with the mounting cavity (130); a rotary control member (400) is provided in the rotary control cavity; and the rotary control member (400) is connected to the valve spool (200).

3. The control valve according to claim 2, wherein a sliding groove (410) is provided on a peripheral wall of the rotary control member (400); the balance member (140) is provided on an inner wall of the rotary control cavity; the balance member (140) abuts against the valve spool (200) via the rotary control member (400); and the balance member (140) is in a sliding fit with the sliding groove (410).

4. The control valve according to claim 3, wherein a mounting groove (430) matched with the balance member (140) is provided on the peripheral wall of the rotary control member (400); the mounting groove (430) is provided along an axial direction of the rotary control cavity; and the mounting groove (430) communicates with the sliding groove (410).

5. The control valve according to claim 4, wherein a stop member (420) is provided in the sliding groove (410); and the stop member (420) is configured to limit a sliding stroke of the balance member (140) in the sliding groove (410) to control the rotational stroke of the valve spool (200).

6. The control valve according to any one of claims 1 to 5, wherein the output passage (120) comprises a first output sub-passage (121) and a second output sub-passage (122); the first output sub-passage (121) is configured to be connected to a bottle body (500); and the second output sub-passage (122) is configured to be connected to a negative pressure source;
the communication passage (210) comprises a first communication sub-passage (211), a second communication sub-passage (212), and a third communication sub-passage (213);
the through hole (310) comprises a first sub-hole (311) and a second sub-hole (312); and
the rotational stroke of the valve spool (200) comprises a first position and a second position; in the first position, the input passage (110), the first sub-hole (311), the first communication sub-passage (211), and the first output sub-passage (121) communicate sequentially, and the first output sub-passage (121), the second communication sub-passage (212), the second sub-hole (312), and the second output sub-passage (122) communicate sequentially; and in the second position, the input passage (110), the first sub-hole (311), the third communication sub-passage (213), the second sub-hole (312), and the second output sub-passage (122) communicate sequentially.

7. The control valve according to claim 6, wherein the communication passage (210) is located above the first output sub-passage (121).

8. The control valve according to claim 7, wherein the valve spool (200) is provided with a sealing surface (220); the sealing surface (220) is configured to isolate the first output sub-passage (121) from the mounting cavity (130); and an output port of the first communication sub-passage (211) and an input port of the second communication sub-passage (212) are located on the sealing surface (220).

9. A specimen collection container, comprising a bottle body (500) and the control valve according to any one of claims 1 to 8, wherein the control valve is detachably connected to the bottle body (500); and the output passage (120) communicates with an interior of the bottle body (500).

10. An endoscope, comprising a handle (600), wherein the handle (600) is provided with a specimen extraction port (610); the endoscope further comprises the control valve according to any one of claims 1 to 8 or the specimen collection container according to claim 9; and the specimen extraction port (610) communicates with the input passage (110).

## Patentansprüche

1. Ein Steuerventil für einen Probenentnahmebehälter, umfassend:
einen Ventilkörper (100), wobei der Ventilkörper (100) mit einem Eingangskanal (110) und einem Ausgangskanal (120) versehen ist; der Ventilkörper (100) im Inneren mit einem Aufnahmehohlraum (130) versehen ist; und der Eingangskanal (110) und der Ausgangskanal (120) jeweils mit dem Aufnahmehohlraum (130) in Verbindung stehen;
eine Hauptdichtung (300), wobei die Hauptdichtung (300) eine ringförmige Struktur ist; die Hauptdichtung (3 00) fest mit einer Innenwand des Aufnahmehohlraums (130) verbunden ist; und
einen Ventilschieber (200), wobei der Ventilschieber (200) zumindest teilweise innerhalb der Hauptdichtung (300) vorgesehen ist; und
zwischen dem Ventilschieber (200) und der Hauptdichtung (300) ein Drehhub besteht; und
der Ventilschieber (200) mit einem Verbindungskanal (210) versehen ist;
**dadurch gekennzeichnet, dass** die Hauptdichtung (300) mit einer Durchgangsöffnung (310) versehen ist, die entsprechend mit dem Eingangskanal (110) in Verbindung steht; und der Verbindungskanal (210) dazu konfiguriert ist, eine Verbindung zwischen der Durchgangsöffnung (310) und dem Ausgangskanal (120) zu ermöglichen; eine dem Ventilschieber (200) benachbarte Seitenwand der Hauptdichtung (300) mit einer ringförmigen Unterdichtung (320) versehen ist; und die Unterdichtung (320) dazu konfiguriert ist, eine abgedichtete Verbindung zwischen dem Verbindungskanal (210) und der Durchgangsöffnung (310) zu ermöglichen;
und
das Steuerventil ferner ein Ausgleichselement (140) umfasst, wobei das Ausgleichselement (140) an dem Ventilkörper (100) vorgesehen ist; und das Ausgleichselement (140) und die Unterdichtung (320) jeweils an zwei Seiten des Ventilschiebers (200) anliegen, um den Ventilschieber (200) während des Drehhubs koaxial mit der Hauptdichtung (300) zu halten.

2. Steuerventil nach Anspruch 1, wobei der Ventilkörper (100) mit einem Drehsteuerhohlraum versehen ist, der mit dem Aufnahmehohlraum (130) in Verbindung steht; ein Drehsteuerelement (400) in dem Drehsteuerhohlraum vorgesehen ist; und das Drehsteuerelement (400) mit dem Ventilschieber (200) verbunden ist.

3. Steuerventil nach Anspruch 2, wobei eine Gleitnut (410) an einer Umfangswand des Drehsteuerelements (400) vorgesehen ist; das Ausgleichselement (140) an einer Innenwand des Drehsteuerhohlraums vorgesehen ist; das Ausgleichselement (140) über das Drehsteuerelement (400) an dem Ventilschieber (200) anliegt; und das Ausgleichselement (140) gleitend in die Gleitnut (410) eingepasst ist.

4. Steuerventil nach Anspruch 3, wobei eine an das Ausgleichselement (140) angepasste Aufnahmenut (430) an der Umfangswand des Drehsteuerelements (400) vorgesehen ist; die Aufnahmenut (430) entlang einer axialen Richtung des Drehsteuerhohlraums vorgesehen ist; und die Aufnahmenut (430) mit der Gleitnut (410) in Verbindung steht.

5. Steuerventil nach Anspruch 4, wobei ein Anschlagelement (420) in der Gleitnut (410) vorgesehen ist; und das Anschlagelement (420) dazu konfiguriert ist, einen Gleithub des Ausgleichselements (140) in der Gleitnut (410) zu begrenzen, um den Drehhub des Ventilschiebers (200) zu steuern.

6. Steuerventil nach einem der Ansprüche 1 bis 5, wobei der Ausgangskanal (120) einen ersten Ausgangsteilkanal (121) und einen zweiten Ausgangsteilkanal (122) umfasst; der erste Ausgangsteilkanal (121) dazu konfiguriert ist, mit einem Flaschenkörper (500) verbunden zu werden; und der zweite Ausgangsteilkanal (122) dazu konfiguriert ist, mit einer Unterdruckquelle verbunden zu werden;
der Verbindungskanal (210) einen ersten Verbindungsteilkanal (211), einen zweiten Verbindungsteilkanal (212) und einen dritten Verbindungsteilkanal (213) umfasst;
die Durchgangsöffnung (310) eine erste Teilöffnung (311) und eine zweite Teilöffnung (312) umfasst; und
der Drehhub des Ventilschiebers (200) eine erste Position und eine zweite Position umfasst; in der ersten Position der Eingangskanal (110), die erste Teilöffnung (311), der erste Verbindungsteilkanal (211) und der erste Ausgangsteilkanal (121) nacheinander in Verbindung stehen und der erste Ausgangsteilkanal (121), der zweite Verbindungsteilkanal (212), die zweite Teilöffnung (312) und der zweite Ausgangsteilkanal (122) nacheinander in Verbindung stehen; und in der zweiten Position der Eingangskanal (110), die erste Teilöffnung (311), der dritte Verbindungsteilkanal (213), die zweite Teilöffnung (312) und der zweite Ausgangsteilkanal (122) nacheinander in Verbindung stehen.

7. Steuerventil nach Anspruch 6, wobei der Verbindungskanal (210) oberhalb des ersten Ausgangsteilkanals (121) angeordnet ist.

8. Steuerventil nach Anspruch 7, wobei der Ventilschieber (200) mit einer Dichtfläche (220) versehen ist; die Dichtfläche (220) dazu konfiguriert ist, den ersten Ausgangsteilkanal (121) von dem Aufnahmehohlraum (130) zu isolieren; und eine Ausgangsöffnung des ersten Verbindungsteilkanals (211) und eine Eingangsöffnung des zweiten Verbindungsteilkanals (212) an der Dichtfläche (220) angeordnet sind.

9. Probenentnahmebehälter, umfassend einen Flaschenkörper (500) und das Steuerventil nach einem der Ansprüche 1 bis 8, wobei das Steuerventil lösbar mit dem Flaschenkörper (500) verbunden ist; und der Ausgangskanal (120) mit einem Innenraum des Flaschenkörpers (500) in Verbindung steht.

10. Endoskop, umfassend einen Handgriff (600), wobei der Handgriff (600) mit einem Probenentnahmeanschluss (610) versehen ist; das Endoskop ferner das Steuerventil nach einem der Ansprüche 1 bis 8 oder den Probenentnahmebehälter nach Anspruch 9 umfasst; und der Probenentnahmeanschluss (610) mit dem Eingangskanal (110) in Verbindung steht.

## Revendications

1. Vanne de commande pour un récipient de collecte d'échantillon, comprenant :
un corps de valve (100), dans laquelle le corps de valve (100) est pourvu d'un passage d'entrée (110) et d'un passage de sortie (120) ; le corps de valve (100) est pourvu, à l'intérieur, d'une cavité de montage (130) ; et le passage d'entrée (110) et le passage de sortie (120) sont en communication avec la cavité de montage (130) séparément ;
un joint d'étanchéité principal (300), dans laquelle le joint d'étanchéité principal (300) est une structure annulaire ; le joint d'étanchéité principal (300) est relié de manière fixe à une paroi interne de la cavité de montage (130) ; et
un tiroir de vanne (200), dans laquelle le tiroir de vanne (200) est ménagé, au moins en partie, à l'intérieur du joint principal (300) ; et
une course de rotation est prévue entre le tiroir de vanne (200) et le joint principal (300) ; et
le tiroir de vanne (200) est pourvu d'un passage de communication (210) ;
**caractérisée en ce que** le joint d'étanchéité principal (300) est pourvu d'un trou traversant (310) en communication avec le passage d'entrée (110) de manière correspondante ; et le passage de communication (210) est configuré pour permettre la communication entre le trou traversant (310) et le passage de sortie (120) ; une paroi latérale du joint principal (300) adjacente au tiroir de vanne (200) est pourvue d'un sous-joint d'étanchéité annulaire (320) ; et le sous-joint d'étanchéité (320) est configuré pour permettre la communication étanche entre le passage de communication (210) et le trou traversant (310) ;
et
la vanne de commande comprend en outre un élément d'équilibrage (140), dans laquelle l'élément d'équilibrage (140) est ménagé sur le corps de vanne (100) ; et l'élément d'équilibrage (140) et le sous-joint d'étanchéité (320) viennent en butée contre deux côtés du tiroir de vanne (200) respectivement pour maintenir le tiroir de vanne (200) de façon coaxiale avec le joint d'étanchéité principal (300) pendant la course de rotation.

2. Vanne de commande selon la revendication 1, dans laquelle le corps de vanne (100) est pourvu d'une cavité de commande rotative en communication avec la cavité de montage (130) ; un élément de commande rotatif (400) est ménagé dans la cavité de commande rotative ; et l'élément de commande rotatif (400) est relié au tiroir de vanne (200).

3. Vanne de commande selon la revendication 2, dans laquelle une rainure de coulissement (410) est ménagée sur une paroi périphérique de l'élément de commande rotatif (400) ; l'élément d'équilibrage (140) est ménagé sur une paroi interne de la cavité de commande rotative ; l'élément d'équilibrage (140) vient en butée contre le tiroir de vanne (200) via l'élément de commande rotatif (400) ; et l'élément d'équilibrage (140) est en ajustement coulissant avec la rainure de coulissement (410).

4. Vanne de commande selon la revendication 3, dans laquelle une rainure de montage (430) adaptée à l'élément d'équilibrage (140) est ménagée sur la paroi périphérique de l'élément de commande rotatif (400) ; la rainure de montage (430) est ménagée le long d'une direction axiale de la cavité de commande rotative ; et la rainure de montage (430) est en communication avec la rainure de coulissement (410).

5. Vanne de commande selon la revendication 4, dans laquelle un élément de butée (420) est ménagé dans la rainure de coulissement (410) ; et l'élément de butée (420) est configuré pour limiter une course de coulissement de l'élément d'équilibrage (140) dans la rainure de coulissement (410) pour commander la course de rotation du tiroir de vanne (200).

6. Vanne de commande selon l'une quelconque des revendications 1 à 5, dans laquelle le passage de sortie (120) comprend un premier sous-passage de sortie (121) et un deuxième sous-passage de sortie (122) ; le premier sous-passage de sortie (121) est configuré pour être raccordé à un corps de bouteille (500) ; et le deuxième sous-passage de sortie (122) est configuré pour être raccordé à une source de pression négative ;
le passage de communication (210) comprend un premier sous-passage de communication (211), un deuxième sous-passage de communication (212) et un troisième sous-passage de communication (213) ;
le trou traversant (310) comprend un premier sous-trou (311) et un deuxième sous-trou (312) ; et
la course de rotation du tiroir de vanne (200) comprend une première position et une deuxième position ; dans la première position, le passage d'entrée (110), le premier sous-trou (311), le premier sous-passage de communication (211) et le premier sous-passage de sortie (121) communiquent en séquence, et le premier sous-passage de sortie (121), le deuxième sous-passage de communication (212), le deuxième sous-trou (312) et le deuxième sous-passage de sortie (122) communiquent en séquence ; et dans la deuxième position, le passage d'entrée (110), le premier sous-trou (311), le troisième sous-passage de communication (213), le deuxième sous-trou (312) et le deuxième sous-passage de sortie (122) communiquent en séquence.

7. Vanne de commande selon la revendication 6, dans laquelle le passage de communication (210) est situé au-dessus du premier sous-passage de sortie (121).

8. Vanne de commande selon la revendication 7, dans laquelle le tiroir de vanne (200) est pourvu d'une surface d'étanchéité (220) ; la surface d'étanchéité (220) est configurée pour isoler le premier sous-passage de sortie (121) de la cavité de montage (130) ; et un port de sortie du premier sous-passage de communication (211) et un port d'entrée du deuxième sous-passage de communication (212) sont situés sur la surface d'étanchéité (220).

9. Récipient de collecte d'échantillon, comprenant un corps de bouteille (500) et la vanne de commande selon l'une quelconque des revendications 1 à 8, dans lequel la vanne de commande est reliée de manière amovible au corps de bouteille (500) ; et le passage de sortie (120) est en communication avec un intérieur du corps de bouteille (500).

10. Endoscope, comprenant une poignée (600), dans lequel la poignée (600) est pourvue d'un port d'extraction d'échantillon (610) ; l'endoscope comprend en outre la vanne de commande selon l'une quelconque des revendications 1 à 8 ou le récipient de collecte d'échantillon selon la revendication 9 ; et le port d'extraction d'échantillon (610) est en communication avec le passage d'entrée (110).
